(19) Europäisches Patentamt
European Patent Office
Office européen des brevets

(11) Publication number: **0 343 801 B1**

(12) **EUROPEAN PATENT SPECIFICATION**

(45) Date of publication of patent specification: **03.08.94**

(51) Int. Cl.[5]: **C07D 471/04**, A61K 31/505, //(C07D471/04,239:00,221:00)

(21) Application number: **89304499.0**

(22) Date of filing: **04.05.89**

The file contains technical information submitted after the application was filed and not included in this specification

(54) **N-(5,6,7,8-tetrahydropyrido(2,3-D)pyrimidin-6-yl-alkanoyl)-glutamic acid derivatives.**

(30) Priority: **25.05.88 US 198201**
**25.05.88 US 198207**

(43) Date of publication of application:
**29.11.89 Bulletin 89/48**

(45) Publication of the grant of the patent:
**03.08.94 Bulletin 94/31**

(84) Designated Contracting States:
**AT BE CH DE ES FR GB GR IT LI LU NL SE**

(56) References cited:
**WO-A-86/05181**

**Medicinal Chemistry, Third Edition, part I, edited by A. Burger pp.72-74, 77**

(73) Proprietor: **THE TRUSTEES OF PRINCETON UNIVERSITY**
**Nassau Street**
**Princeton New Jersey 08544(US)**

Proprietor: **ELI LILLY AND COMPANY**
**Lilly Corporate Center**
**Indianapolis Indiana 46285(US)**

(72) Inventor: **Taylor, Edward C.**
**288 Western Way, Princeton**
**New Jersey 08544(US)**
Inventor: **Shih, Chuan**
**8521 East Scarsdale Drive**
**Indianapolis, Indiana 46256(US)**

(74) Representative: **Daley, Michael John et al**
**F.J. CLEVELAND & COMPANY**
**40/43 Chancery Lane**
**London, WC2A 1JO (GB)**

EP 0 343 801 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention pertains to the individual diastereomers and to the diastereomeric mixture of glutamic acid derivatives of the formula:

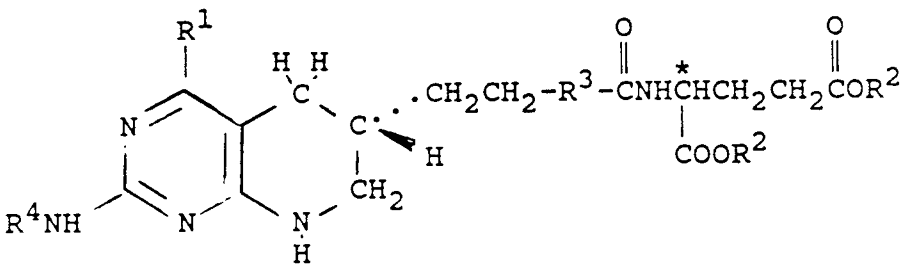

IA

or

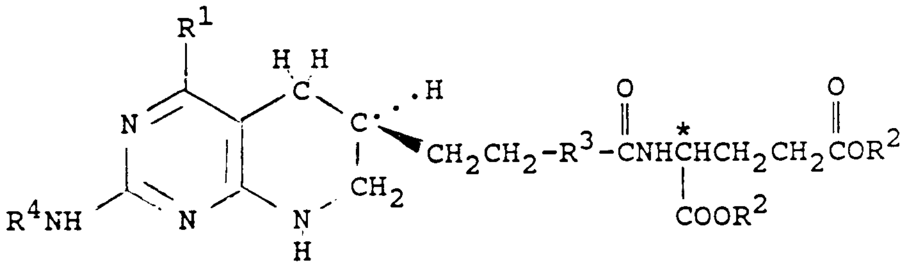

IB

in which:

- $R^1$ is -OH or $-NH_2$;
- $R^2$ is hydrogen or a carboxy protecting group;
- $R^3$ is a carbon-carbon bond, alkylene of 1 to 4 carbon atoms, cyclohexylene, or

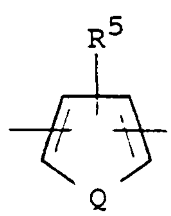

;

- $R^4$ is hydrogen or an amino protecting group;
- Q is -S- or -O-;
- $R^5$ is hydrogen, chloro or fluoro; and

the configuration about the carbon atom designated * is S.

The compounds of Formula IA and IB have an inhibitory effect on one or more enzymes which utilize folic acid, and in particular metabolic derivatives of folic acid, as a substrate. The compounds thus can be used, alone or in combination, to inhibit the growth of those neoplasms which otherwise depend upon the enzymes so inhibited.

The invention also pertains to the pharmaceutically acceptable salts of the compounds of Formula IA and IB, to processes for the preparation of these compounds and their salts and to pharmaceutical compositions containing these compounds or their salts.

The term alkylene as used herein denotes a straight or branched divalent aliphatic group of from 1 to 4 carbon atoms including methylene, ethylene, trimethylene, tetramethylene, 1,1-propylidene, 2,2-propylidene, 1,2-propanediyl, 2,3-butanediyl, etc. Analogously, cyclohexylene denotes a divalent cycloalkane group of 6 carbon atoms including 1,2-cyclohexylene, 1,3-cyclohexylene, and 1,4-cyclohexylene.

The protecting groups designated by $R^2$ and $R^4$ and utilized herein denote groups which generally are not found in the final therapeutic compounds but which are intentionally introduced during a portion of the synthesis to protect a group which otherwise might react in the course of chemical manipulations, thereafter being removed at a later stage of the synthesis. Since compounds bearing such protecting groups thus are of importance primarily as chemical intermediates (although some derivatives also exhibit biological activity), their precise structure is not critical. Numerous reactions for the formation and removal of such protecting groups are described in a number of standard works including, for example, "Protective Groups in Organic Chemistry", Plenum Press, London and New York, 1973; Greene, Th. W. "Protective Groups in Organic Synthesis", Wiley, New York, 1981; "The Peptides", Vol. I, Schröder and Lubke, Academic Press, London and New York, 1965; "Methoden der organischen Chemie", Houben-Weyl, 4th Edition, Vol.15/I, Georg Thieme Verlag, Stuttgart 1974.

A carboxy group can be protected as an ester group which is selectively removable under sufficiently mild conditions not to disrupt the desired structure of the molecule, especially a lower alkyl ester such as methyl or ethyl and particularly one which is branched at the 1-position such as t.-butyl; and such lower alkyl ester substituted in the 1- or 2-position with (i) lower alkoxy, such as for example, methoxymethyl, 1-methoxyethyl, and ethoxymethyl, (ii) lower alkylthio, such as for example methylthiomethyl and 1-ethylthioethyl; (iii) halogen, such as 2,2,2-trichloroethyl, 2-bromoethyl, and 2-iodoethoxycarbonyl; (iv) one or two phenyl groups each of which can be unsubstituted or mono-, di-or tri-substituted with, for example lower alkyl such as tert.-butyl, lower alkoxy such as methoxy, hydroxy, halo such as chloro, and nitro, such as for example, benzyl, 4-nitrobenzyl, diphenylmethyl, di-(4-methoxyphenyl)methyl; or (v) aroyl, such as phenacyl. A carboxy group can also be protected in the form of an organic silyl group such as tri-lower alkylsilyl, as for example trimethylsilyloxycarbonyl.

Amino groups similarly can be protected as an amide utilizing an acyl group which is selectively removable under mild conditions, especially formyl, a lower alkanoyl group which is branched at the 1-position, particularly tertiary alkanoyl such as pivaloyl, or a lower alkanoyl group which is substituted in the 1-position, as for example trifluoroacetyl.

A first group of preferred compounds are those wherein $R^1$ is -OH and each of $R^2$ and $R^4$ is hydrogen. A first preferred subgroup within this group are those compounds in which $R^3$ is a carbon-carbon bond, methylene, ethylene, trimethylene, tetramethylene, or 1,4-cyclohexylene. Preferred species within this subgroup include the (R,S) and (S,S) diastereomers of N-[3-(2-amino-4-hydroxy-5,6,7,8-tetrahydropyrido-[2,3-d]-pyrimidin-6-yl)propioyl]-L-glutamicacid;N-[4-(2-amino-4-hydroxy-5,6,7,8-tetrahydropyrido[2,3-d]-pyrimidin-6-yl)butyryl]-L-glutamic acid; N-[5-(2-amino-4-hydroxy-5,6,7,8-tetrahydropyrido[2,3-d]pyrimidin-6-yl)-pentanoyl]-L-glutamic acid; N-[6-(2-amino-4-hydroxy-5,6,7,8-tetrahydropyrido[2,3-d]pyrimidin-6-yl)hexanoyl]-L-glutamic acid; N-[7-(2-amino-4-hydroxy-5,6,7,8-tetrahydropyrido[2,3-d]pyrimidin-6-yl)heptanoyl]-L-glutamic acid; and N-[4-(2-{2-amino-4-hydroxy-5,6,7,8-tetrahydropyrido[2,3-d]pyrimidin-6-yl}ethyl)cyclohex-1-ylcarbonyl]-L-glutamic acid.

A second preferred subclass are those compounds in which $R^3$ is:

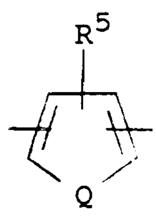

where $R^5$ is hydrogen and Q is -S-. Preferred species within this subgroup include the (R,S) and (S,S) diastereomers of N-[5-(2-{2-amino-4-hydroxy-5,6,7,8-tetrahydropyrido[2,3-d]-pyrimidin-6-yl)ethyl)thien-2-ylcarbony]-L-glutamic acid; N-[5-(4-{2-amino-4-hydroxy-5,6,7,8-tetrahydropyrido[2,3-d]pyrimidin-6-yl}ethyl)-thien-2-ylcarbony]-L-glutamic acid; N-[5-(2-{2-amino-4-hydroxy-5,6,7,8-tetrahydropyrido[2,3-d]pyrimidin-6-yl}ethyl)thien-3-ylcarbony]-L-glutamic acid; N-[4-(2-{2-amino-4-hydroxy-5,6,7,8-tetrahydropyrido[2,3-d]-pyrimidin-6-yl}ethyl)thien-3-ylcarbony]-L-glutamic acid; N-[3-(2-{2-amino-4-hydroxy-5,6,7,8-tetrahydropyrido[2,3-d]pyrimidin-6-yl}ethyl)thien-2-ylcarbony]-L-glutamic acid; N-[2-(2-{2-amino-4-hydroxy-5,6,7,8-tetrahydropyrido[2,3-d]pyrimidin-6-yl}ethyl)thien-3-ylcarbony]-L-glutamic acid; and N-[5-(2-{2-amino-4-hydroxy-5,6,7,8-tetrahydropyrido[2,3-d]pyrimidin-6-yl}ethyl)fur-2-ylcarbony]-L-glutamic acid.

The compounds of the present invention often can be employed advantageously in the form of a pharmaceutically acceptable salt. Such forms, including hydrates thereof, are often crystalline and advantageous for forming solutions or formulating pharmaceutical compositions. Pharmaceutically acceptable salts

with bases include those formed from the alkali metals, alkaline earth metals, non-toxic metals, ammonium, and mono-, di- and trisubstituted amines, such as for example the sodium, potassium, lithium, calcium, magnesium, aluminium, zinc, ammonium, trimethylammonium, triethanolammonium, pyridinium, and substituted pyridinium salts. The mono and disodium salts, particularly the disodium salt, are advantageous.

The compounds of this invention in which $R^3$ is alkylene or

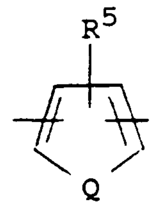

can be prepared through catalytic hydrogenation of a compound of the formula:

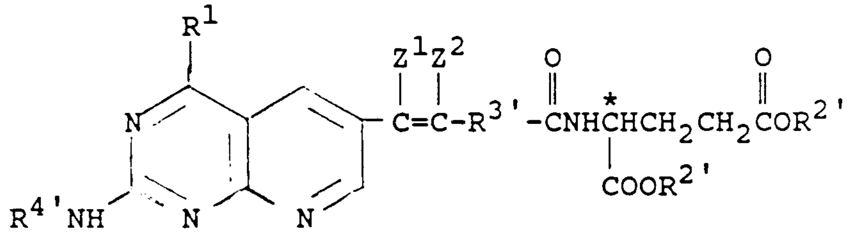

II

in which:

$Z^1$ and $Z^2$ taken individually are each hydrogen or taken together are a carbon-carbon bond;
$R^1$ is as herein defined;
$R^{2'}$ is a carboxy protecting group;
$R^{3'}$ is a carbon-carbon bond alkylene of 1 to 4 carbon atoms, or

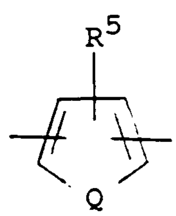

;

and
$R^{4'}$ is an amino protecting group.

Suitable hydrogenation catalysts include noble metals and noble metal oxides such as palladium or platinum oxide, rhodium oxide, and the foregoing on a support such as carbon or calcium oxide.

There is obtained a mixture of diastereomers of Formulas IA and IB in which $R^{2'}$ is a carboxy protecting group, and $R^{4'}$ is an amino protecting group. These protecting groups can then be removed through acidic or basic hydrolysis, as for example with sodium hydroxide, to yield the compounds of Formula I in which each of $R^2$ and $R^4$ is hydrogen.

Compounds of Formula II can be prepared utilizing procedures analogous to those described in European Patent Application 87308921.3. Thus in a first embodiment, a compound of the formula:

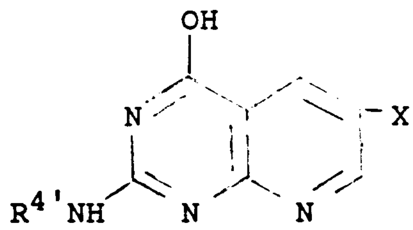

III

in which X is bromo or iodo and $R^{4'}$ is as herein defined, is allowed to react with an unsaturated compound of the formula:

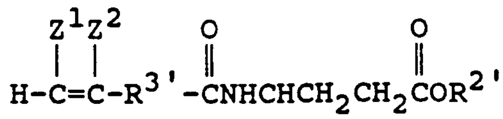

IV

in which $Z^1$, $Z^2$, $R^{2'}$, and $R^{3'}$ are as herein defined, in the presence of a palladium/trisubstituted phosphine catalyst of the type described by Sakamoto, Synthesis, 1983, 312 et seq.

The compounds of this invention in which $R^3$ is cyclohexylene can be prepared through initial catalytic hydrogenation of a compound of the formula:

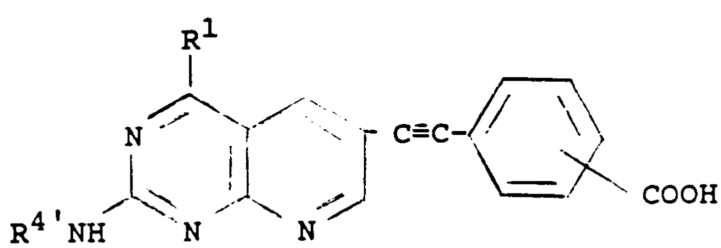

V

in which $R^{4'}$ is an amino protecting group and $R^1$ is a herein defined. Compounds of Formula V in which $R^1$ is -OH are prepared analogously to the methods described in European Patent Application 87308921.3 and the corresponding compounds in which $R^1$ is $-NH_2$ are generated therefrom in the manner described above.

Suitable catalysts for the hydrogenation of compounds of Formula V include noble metals and noble metal oxides such as palladium or platinum oxide. Thus obtained is a compound of the formula:

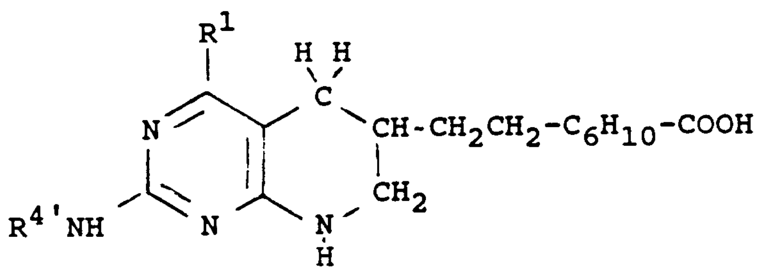

VI

Compounds of Formula VI then are coupled with a protected glutamic acid derivative of the formula:

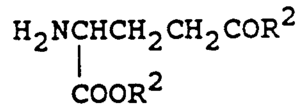

VII

in which $R^{2'}$ is a carboxy protecting group, in the manner generally described in PCT application WO 86/05181, utilizing conventional condensation techniques for forming peptide bonds, such as activation of the carboxy group through formation of a mixed anhydride, treatment with DCC, or use of diphenylchlorophosphonate. Protecting groups designated by $R^{2'}$ and $R^{4'}$ then are removed in the manner described above.

In a third embodiment, compounds of Formula II can be prepared utilizing procedures analogous to those described in European Patent Application 87308921.3. Thus an unsaturated compound of the formula:

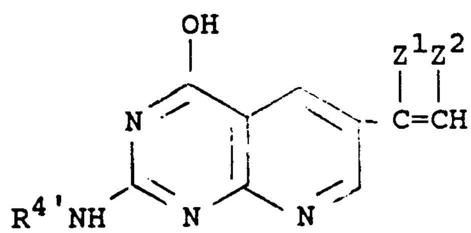

VIII

in which $Z^1$, $Z^2$, and $R^{4'}$ are as herein defined, is allowed to react with compound of the formula:

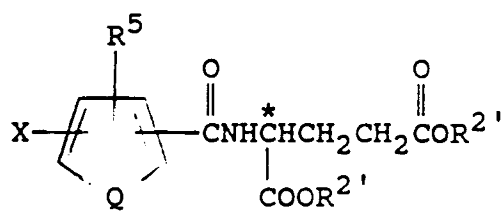

IX

in which X is bromo or iodo and $R^{2'}$, $R^5$, and Q are as herein defined, in the presence of a palladium/trisubstituted phosphine catalyst of the type described by Sakamoto, Synthesis, 1983, 312 et seq.

There is obtained according to the foregoing processes a compound of Formula II in which $R^1$ is -OH. When a compound in which $R^1$ is $-NH_2$ is desired, this product can be treated with 1,2,4-triazole and (4-chlorophenyl)dichlorophosphate and the product of this reaction then treated with concentrated ammonia.

Compounds of Formula IX are prepared by coupling a compound of the formula:

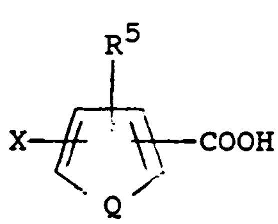

X

in which X, $R^5$, and Q are as herein defined, with a protected glutamic acid derivative of formula VII in the manner described above utilizing conventional condensation techniques for forming peptide bonds

The mixture of the individual diastereomers depicted by Formulas IA and IB can be used therapeutically as such or can be separated mechanically as by chromatography. Alternatively, the individual diastereomers can be separated by forming diastereomeric salts with a chiral acid such as the individual enantiomers of 10-camphorsulfonic acid, camphoric acid, alpha-bromocamphoric acid, methoxyacetic acid, tartaric acid, diacetyltartaric acid, malic acid, pyrrolidone-5-carboxylic acid, and the like, and then freeing one or both of the individual diastereomeric bases, optionally repeating the process, so as obtain either or both substantially free of the other; i.e., in a form having an optical purity of >95%. This separation can be effected before or after removal of any protecting groups.

As noted, the compounds of this invention have an effect on one or more enzymes which utilize folic acid, and in particular metabolic derivatives of folic acid, as a substrate. The compounds can be used, under the supervision of qualified professionals, to inhibit the growth of neoplasms including choriocarcinoma, leukemia, adenocarcinoma of the female breast, epidermid cancers of the head and neck, squamous or small-cell lung cancer, and various lymphosarcomas. The compounds can also be used to treat mycosis fungoides and psoriasis.

The compounds can be administered orally but preferably are administered parenterally, alone or in combination with other therapeutic agents including other anti-neoplastic agents, steroids, etc., to a mammal suffering from neoplasm and in need of treatment. Parenteral routes of administration include intramuscular, intrathecal, intravenous and intra-arterial. Dosage regimens must be titrated to the particular neoplasm, the condition of the patient, and the response but generally doses will be from about 10 to about 100 mg/day for 5-10 days or single daily administration of 250-500 mg, repeated periodically; e.g. every 14 days. While having a low toxicity as compared to other antimetabolites now in use, a toxic response often can be eliminated by either or both of reducing the daily dosage or administering the compound on alternative days or at longer intervals such as every three days. Oral dosage forms include tablets and capsules containing from 1-10 mg of drug per unit dosage. Isotonic saline solutions containing 20-100 mg/ml can be used for parenteral administration.

The following examples will serve to further illustrate the invention. In the NMR data, "s" denotes singlet, "d" denotes doublet, "t" denotes triplet, "q" denotes quartet, "m" denotes multiplet, and "br" denotes a broad peak.

EXAMPLE 1

Dimethyl N-[6-(2-Pivaloylamino-4-hydroxypyrido[2,3-d]pyrimidin-6-yl)hex-5-ynoyl]-L-glutamate

A mixture of 1.94g. (6 mmol.) of 2-pivaloylamino-4-hydroxy-6-bromopyrido[2,3-d]pyrimidine, 1.6g. (6 mmol.) of dimethyl N-(hex-5-ynoyl)-L-glutamate, 0.11g. of palladium chloride, 0.32g. of triphenylphosphine, 0.05g. of cuprous iodide, and 2.6mL. of triethylamine in 150mL. of acetonitrile was heated at reflux for 3 hours and then cooled to ambient temperature. The solvent was removed under reduced pressure and the residue chromatographed on silica gel with 1:9 methanol: methylene chloride to yield dimethyl N-[6-(2-pivaloylamino-4-hydroxypyrido[2,3-d]pyrimidin-6-yl))hex-5-ynoyl]-L-glutamate, m.p. 159-160°C. Anal. Calc. for $C_{25}H_{31}N_5O_7$: C, 58.47; H, 6.08; N, 13.64. Found: C, 58.23; H, 5.97; N, 13.43.

In as similar fashion by substituting an equivalent amount of dimethyl N-(pent-4-ynoyl)-L-glutamate for dimethyl N-(hex-5-ynoyl)-L-glutamate in the foregoing procedure, there can be obtained dimethyl N-[5-(2-pivaloylamino-4-hydroxypyrido[2,3-d]pyrimidin-6-yl)pent-4-ynoyl]-L-glutamate. In a representative experiment, the following physical constants were obtained for this compound: m.p. 162-163° C. Anal. Calc. for $C_{24}H_{29}N_5O_7$: C, 57.71; H, 5.84; N, 14.02. Found: C, 57.94; H, 5.72; N, 13.99.

Likewise from dimethyl N-acryloyl-L-glutamate and dimethyl N(but-3-ynoyl)-L-glutamate there are respectively obtained dimethyl N-[3-(2-pivaloylamino-4-hydroxypyrido[2,3-d]pyrimidin-6-yl)acryloyl]-L-glutamate and dimethyl N-[4-(2-pivaloylamino-4-hydroxypyrido[2,3-d]pyrimidin-6-yl)but-3-ynoyl]-L-glutamate.

By substituting an equivalent amount of dimethyl N-(hept-6-enoyl)-L-glutamate for dimethyl N-(hex-5-ynoyl)-L-glutamate in the foregoing procedure, there can be obtained dimethyl N-[7-(2-pivaloylamino-4-hydroxypyrido[2,3-d]pyrimidin-6-yl)hept-6-enoyl]-L-glutamate. In a representative experiment, the following physical constants were obtained for this compound: m.p. 79-82°C. Anal. Calc. for $C_{26}H_{35}N_5O_7$: C, 58.01; H, 6.82; N, 13.53. Found: C, 58.47; H, 6.48; N, 12.80.

Dimethyl N-(hex-5-ynoyl)-L-glutamate can be obtained by allowing hex-5-ynoic acid chloride (obtained by treating hex-5-ynoic acid with thionyl chloride) to react with dimethyl L-glutamate in the presence of an acid acceptor such as triethylamine. Hex-5-ynoic acid in turn can be prepared, for example, by alkaline

hydrolysis of 5-cyanopent-1-yne.

Dimethyl N-acryloyl-L-glutamate, dimethyl N-(pent-4-ynoyl)-L-glutamate, dimethyl N-(but-3-ynoyl)-L-glutamate, and dimethyl N-(hept-6-enoyl)-L-glutamate are obtained similarly from the acid chlorides of acrylic acid, pent-4-ynoic acid, but-3-ynoic acid, and hept-6-enoic acid, respectively, and dimethyl L-glutamate.

EXAMPLE 2

Dimethyl N-[6-(2-Pivaloylamino-4-hydroxy-5,6,7,8-tetrahydropyrido[2,3-d]pyrimidin-6-yl)hexanoyl]-L-glutamate

To a solution of 1.0g of dimethyl N-[6-(2-pivaloylamino-4-hydroxypyrido[2,3-d]pyrimidin-6-yl)hex-5-ynoyl]-L-glutamate in 20mL. of glacial acetic acid were added 300 mg. of platinum oxide. The mixture was hydrogenated under one atmosphere pressure with agitation for 4 hours, the catalyst removed by filtration, and the filtrate concentrated under reduced pressure. Chromatography on silica gel eluting with 1:19 methanol:chloroform yielded 0.84g. (82.7%) of dimethyl N-[6-(2-pivaloylamino-4-hydroxy-5,6,7,8-tetrahydropyrido[2,3-d]pyrimidin-6-yl)hexanoyl]-L-glutamate, m.p. 162-166°C. Anal. Calc. for $C_{25}H_{39}N_5O_7$: C, 57.57; H, 7.54; N, 13.43. Found: C, 57.31; H, 7.25; N, 14.15.

Similarly prepared from dimethyl N-[5-(2-pivaloylamino-4-hydroxypyrido[2,3-d]pyrimidin-6-yl)pent-4-ynoyl]-L-glutamate is dimethyl N-[5-(2-pivaloylamino-4-hydroxy-5,6,7,8-tetrahydropyrido[2,3-d]pyrimidin-6-yl)pentanoyl]-L-glutamate. In a representative experiment, the following physical constants were obtained for this compound: m.p. 151-159°C; Anal. Calc. for $C_{24}H_{37}N_5O_7$: C, 56.79; H, 7.35; N, 13.80. Found: C, 57.06; H, 7.22; N, 13.86.

Likewise dimethyl N-[3-(2-pivaloylamino-4-hydroxy-5,6,7,8-tetrahydropyrido[2,3-d]pyrimidin-6-yl)-propionyl]-L-glutamate and dimethyl N-[4-(2-pivaloylamino-4-hydroxy-5,6,7,8-tetrahydropyrido[2,3-d]-pyrimidin-6-yl)butyryl]-L-glutamate are prepared from N-[3-(2-pivaloylamino-4-hydroxypyrido[2,3-d]-pyrimidin-6-yl)acryloyl]-L-glutamate and dimethyl N-[4-(2-pivaloylamino-4-hydroxypyrido[2,3-d]pyrimidin-6-yl)but-3-ynoyl]-L-glutamate, respectively.

By utilizing an equivalent amount of dimethyl N-[7-(2-pivaloylamino-4-hydroxypyrido[2,3-d]pyrimidin-6-yl)hept-6-enoyl]-L-glutamate, there is obtained dimethyl N-[7-(2-pivaloylamino-4-hydroxy-5,6,7,8-tetrahydropyrido[2,3-d]pyrimidin-6-yl)heptanoyl]-L-glutamate In a representative experiment, the following physical constants were obtained for this compound: m.p. 152-160°C. Anal. Calc. for $C_{26}H_{41}N_5O_7$: C, 58.30; H, 7.72; N, 13.08. Found: C, 58.51; H, 7.61; N, 12.87.

EXAMPLE 3

Diethyl N-[4-{2-(2-Pivaloylamino-4-hydroxy-5,6,7,8-tetrahydropyrido[2,3-d]pyrimidin-6-yl)ethyl}cyclohex-1-yl]-L-glutamate

A. Five grams of 2-pivaloylamino-4-hydroxy-6-(4-carboxyphenylethynyl)pyrido[2,3-d]pyrimidine (described in European Patent Application 87308921.3) and 1.60g. of platinum oxide in 200mL. of trifluoroacetic acid were hydrogenated in a Parr apparatus at ambient temperature and 60 psi for 24 hours. Removal of the catalyst by filtration and concentration of the filtrate yielded 2-pivaloylamino-4-hydroxy-6-[2-(4-carboxycyclohex-1-yl)ethyl]-5,6,7,8-tetrahydropyrido[2,3-d]pyrim idine as a white solid which was further purified by column chromatography (Waters 200) eluting with 1:9 methanol:dichloromethane. m.p. 218-234°C. Anal. Calc. for $C_{21}H_{32}N_4O_4$: C, 63.35; H, 7.97; N, 13.85. Found: C, 63.30; H, 7.48; N, 13.57.

B. To a solution of 2.40g. of 2-pivaloylamino-4-hydroxy-6-[2-(4-carboxycyclohex-1-yl)ethyl]-5,6,7,8-tetrahydropyrido[2,3-d]pyrimidine in 50mL. of dry N-methylpyrrolidone and 2.25mL. of N-methylmorpholine were added 2.75g. of phenyl N-phenylphosphoaminochloridate. This mixture was stirred at 0°C under nitrogen for 45 minutes and 2.45g. of diethyl L-glutamate were then added and stirring at ambient temperatures then was continued for 72 hours. The solvent was removed by distillation at 0.2mm/60°C and the residue chromatographed on silica gel, eluting with 1:19 methanol:chloroform to yield 1.67g. (48%) of diethyl N-[4-{2-(2-pivaloylamino-4-hydroxy-5,6,7,8-tetrahydropyrido[2,3-d]pyrimidin-6-yl)-ethyl}cyclohex-1-yl]-L-glutamate, m.p. 160-170°C. Anal. Calc. for $C_{30}H_{47}N_5O_7$: C, 61.10; H, 8.03; N, 11.87. Found: C, 61.38; H, 7.90; N, 11.84.

EXAMPLE 4

N-[6-(2-Amino-4-hydroxy-5,6,7,8-tetrahydropyrido[2,3-d]pyrimidin-6-yl)hexanoyl]-L-glutamic Acid

A solution of 0.5g. of dimethyl N-[6-(2-pivaloylamino-4-hydroxy-5,6,7,8-tetrahydropyrido[2,3-d]pyrimidin-6-yl)hexanoyl]-L-glutamate in 75mL. of 1.0 N aqueous sodium hydroxide is stirred at room temperature for 120 hours and the pH then adjusted to 7.0 through the careful addition of 5.0 N hydrochloric acid. Water was removed under reduced pressure and the concentrated solution then cooled in an ice-bath. The solid which formed was collected by filtration and dried at 80°C to yield N-[6-(2-amino-4-hydroxy-5,6,7,8-tetrahydropyrido[2,3-d]pyrimidin-6-yl)hexanoyl]-L-glutamic acid, m.p. foaming at 135°C, melting at 180-195°C. NMR (DMSO-$d_6$ 300 MHz) delta: 9.80 (s, br, 1H), 7.90 (s, J = 8 Hz, 1H), 6.20 (s, 1H), 5.95 (s, 2H), 4.08 (m, 1H), 3.10 (m, 1H), 2.63 (t, J = 10 Hz, 1H), 2.40 (m, 1H), 2.20 (t, J = 8 Hz, 2H), 2.05 (t, J = 8 Hz, 2H), 1.84 (m, 1H), 1.70 (m, 2H), 1.55 (m, 3H), 1.20 (m, 6H).

Similarly prepared from dimethyl N-[5-(2-pivaloylamino-4-hydroxy-5,6,7,8-tetrahydropyrido[2,3-d]-pyrimidin-6-yl)pentanoyl]-L-glutamate is N-[5-(2-amino-4-hydroxy-5,6,7,8-tetrahydropyrido[2,3-d]pyrimidin-6-yl)pentanoyl]-L-glutamic acid. In a representative experiment, the following physical constants were obtained for this compound: m.p. 144°C (softening and foaming); NMR (DMSO-$d_6$ 300 MHz) delta: 9.70 (s, br, 1H), 8.01 (d, J = 6 Hz, 1H), 6.17 (s, 1H), 5.88 (s, 2H), 4.13 (m, 1H), 3.17 (m, 1H), 2.68 (t, J = 6 Hz, 1H), 2.42 (m, 1H), 2.22 (t, J = 5 Hz, 2H), 2.07 (t, J = 5 Hz, 2H), 1.88 (m, 1H), 1.70 (m, 2H), 1.44 (m, 3H), 1.24 (m, 4H);

N-[3-(2-Amino-4-hydroxy-5,6,7,8-tetrahydropyrido[2,3-d]pyrimidin-6-yl)propionyl]-L-glutamic acid and N-[4-(2-amino-4-hydroxy-5,6,7,8-tetrahydropyrido[2,3-d]pyrimidin-6-yl)butyryl]-L-glutamic acid are prepared analogously from dimethyl N-[3-(2-pivaloylamino-4-hydroxy-5,6,7,8-tetrahydropyrido[2,3-d]pyrimidin-6-yl)-propionyl]-L-glutamate and dimethyl N-[4-(2-pivaloylamino-4-hydroxy-5,6,7,8-tetrahydropyrido[2,3-d]-pyrimidin-6-yl)butyryl]-L-glutamate, respectively.

In a similar fashion, N-[7-(2-Amino-4-hydroxy-5,6,7,8-tetrahydropyrido[2,3-d]pyrimidin-6-yl)heptanoyl]-L-glutamic acid is prepared from dimethyl N-[7-(2-pivaloylamino-4-hydroxy-5,6,7,8-tetrahydropyrido[2,3-d]-pyrimidin-6-yl)heptanoyl]-L-glutamate. In a representative experiment, the following physical constants were obtained for this compound: m.p. 185-195°C (foaming); NMR (DMSO-$d_6$ 300 MHz) delta: 9.68 (s, br, 1H), 8.01 (d, J = 10 Hz, 1H), 6.20 (s, 1H), 5.88 (s, 2H), 4.14 (m, 1H), 3.10 (m, 1H), 2.66 (t, J = 9 Hz, 1H), 2.35 (m, 1H), 2.20 (t, J = 5 Hz, 2H), 2.05 (t, J = 5 Hz, 2H), 1.89 (m, 1H), 1.67 (m, 2H), 1.45 (m, 3H), 1.20 (m, 8H).

Analogously by employing diethyl N-[4-{2-(2-pivaloylamino-4-hydroxy-5,6,7,8-tetrahydropyrido[2,3-d]-pyrimidin-6-yl)ethyl}cyclohex-1-yl]-L-glutamate, there is obtained N-[4-{2-(2-amino-4-hydroxy-5,6,7,8-tetrahydropyrido[2,3-d]pyrimidin-6-yl)ethyl}cyclohex-1-yl]-L-glutamic acid. In a representative experiment, the following physical constants were obtained for this compound: m.p. 210-225°C. NMR (DMSO-$d_6$ 300 MHz) delta: 9.70 (s, br, 1H), 7.90 (m, 1H), 6.23 (s, 1H), 5.92 (s, 2H), 4.17 (m, 1H), 3.18 (m, 1H), 2.72 (t, J = 9 Hz, 1H), 2.45 (m, 1H), 2.27 (t, J = 6 Hz, 2H), 2.09 (m, 1H), 1.96 (m, 1H), 1.74 (m, 3H), 1.45 (m, 5H), 1.27 (m, 8H).

EXAMPLE 5

Dimethyl N-[5-(2-Pivaloylamino-4-hydroxypyrido[2,3-d]pyrimidin-6-yl}ethynyl)thien-2ylcarbonyl]-L-glutamate

A. A solution of 1.50g. (7.25 mmol.) of 5-bromo-2-thiophenecarboxylic acid, several drops of dimethylformamide, and 2.21mL. of thionyl chloride was heated at reflux for 2.5 hours. The solvent was removed under reduced pressure and the residue redissolved in 10mL. of dry methylene chloride. This solution was added dropwise to an ice-cooled solution of 1.62g. of dimethyl L-glutamate hydrochloride, 10 mg. of dimethylaminopyridine, and 2.11mL. of triethylamine in 25mL. of dry methylene chloride. When the addition was complete, the mixture was stirring at ambient temperatures for 12 hours and then diluted with water and extracted with methylene chloride. The organic extracts were washed with 1.0 N hydrochloric acid and saturated sodium bicarbonate solution and then evaporated to yield 2.71g. (100%) of dimethyl N-[5-bromothien-2-yl-carbonyl]-L-glutamate as a viscous oil which can be used in the following procedure without further purification.

Dimethyl N-[5-bromofur-2-ylcarbonyl]-L-glutamate, also a viscous oil, is prepared analogiusly from 5-bromo-2-furancarboxylic acid.

Dimethyl N-(5-bromothien-3-ylcarbonyl)-L-glutamate can be prepared in a similar fashion from 5-bromo-3-thienylcarboxylic acid.

B. A mixture of 1.70g. (6.32 mmol.) of 2-pivaloylamino-4-hydroxy-6-ethynylpyrido[2,3-d]pyrimidine, 2.30g. (6.32 mmol.) of dimethyl N-(5-bromothien-2-yl-carbonyl)-L-glutamate, 44mg. of palla-dium chloride,

130mg. of triphenylphosphine, 25mg. of cuprous iodide, and 1.13mL. of triethylamine in 30mL. of acetonitrile was heated at reflux for 3 hours and then cooled to ambient temperature. The solvent was removed under reduced pressure and the residue column chromatographed (Waters 500) eluting with 1:19 methanol:methylene chloride to yield dimethyl N-[5-(2-pival-oylamino-4-hydroxypyrido[2,3-d]-pyrimidin-6-yl}ethynyl)thien-2yl-carbonyl]-L-glutamate, m.p. 228-230°C. Anal. Calc. for $C_{26}H_{27}N_5O_7S$: C, 56.41; H, 4.92; N, 12.65; S, 5.79. Found: C, 56.64; H, 4.77; N, 12.88; S, 5.58.

In as similar fashion by substituting an equivalent amount of dimethyl N-(5-bromofur-2-ylcarbonyl)-L-glutamate for dimethyl N-(5-bromothien-2-ylcarbonyl)-L-glutamate in the foregoing procedure, there can be obtained dimethyl N-[5-(2-pivaloylamino-4-hydroxypyrido[2,3-d]pyrimidin-6-yl}ethynyl)fur-2ylcarbonyl]-L-glutamate. In a representative experiment, the following physical constants were obtained for this compound: m.p. 135°C (darkens), 181°C (decomposition). Anal. Calc. for $C_{26}H_{25}N_5O_8$: C, 58.32; H, 4.71; N, 13.08. Found: C, 58.58; H, 4.92; N, 13.11.

Likewise by substituting an equivalent amount of dimethyl N-(5-bromothien-3-ylcarbonyl)-L-glutamate for dimethyl N-(5-bromothien-2-ylcarbonyl)-L-glutamate in the foregoing procedure, there can be obtained dimethyl N-[5-(2-pivaloylamino-4-hydroxypyrido[2,3-d]pyrimidin-6-yl}ethynyl)thien-3-ylcarbonyl]-L-glutamate.

EXAMPLE 6

Dimethyl N-[5-(2-{2-Pivaloylamino-4-hydroxy-5,6,7,8-tetrahydropyrido[2,3-d]pyrimidin-6-yl}ethyl)thien-2-ylcarbonyl]-L-glutamate

To a solution of 0.5g. of dimethyl N-[5-(2-pivaloylamino-4-hydroxypyrido[2,3-d]pyrimidin-6-yl}ethynyl)-thien-2ylcarbonyl]-L-glutamate in 30mL. of glacial acetic acid were added 1.5g. of 5% palladium on carbon. The mixture was hydrogenated under one atmosphere pressure with agitation for 18 hours, the catalyst removed by filtration, and the filtrate concentrated under reduced pressure. Chromatography on silica gel eluting with 1:19 methanol:chloroform yielded 100mg. (19.7%) of dimethyl N-[5-(2-{2-pivaloylamino-4-hydroxypyrido[2,3-d]pyrimidin-6-yl}ethyl)thien-2-ylcarbonyl]-L-glutamate, and 204mg. (40.2%) of the desired dimethyl N-[5-(2-{2-pivaloylamino-4-hydroxy-5,6,7,8-tetrahydropyrido[2,3-d]pyrimidin-6-yl}ethyl)thien-2-ylcarbonyl]-L-glutamate, m.p. 152-160°C. Anal. Calc. for $C_{26}H_{35}N_5O_7S$: C, 55.60; H, 6.28; N, 12.47. Found: C, 55.76; H, 6.23; N, 12.26. The partially hydrogenated by-product can be rehydrogenated to yield additional final product.

Similarly prepared from dimethyl N-[5-(2-pivaloylamino-4-hydroxypyrido[2,3-d]pyrimidin-6-yl}ethynyl)-fur-2ylcarbonyl]-L-glutamate is dimethyl N-[5-(2-{2-pivaloylamino-4-hydroxy-5,6,7,8-tetrahydropyrido[2,3-d]-pyrimidin-6-yl}ethyl)fur-2-ylcarbonyl]-L-glutamate. In a representative experiment, the following physical constants were obtained for this compound: m.p. 155-162°C; Anal. Calc. for $C_{26}H_{33}N_5O8$: C, 57.45; H, 6.12; N, 12.88. Found: C, 57.22; H, 6.08; N, 12.66.

Likewise dimethyl N-[5-(2-{2-pivaloylamino-4-hydroxy-5,6,7,8-tetrahydropyrido[2,3-d]pyrimidin-6-yl}ethyl)thien-3-ylcarbonyl]-L-glutamate can be prepared from dimethyl N-[5-(2-pivaloylamino-4-hydroxypyrido[2,3-d]pyrimidin-6-ylethynyl)thien-3-ylcarbonyl]-L-glutamate.

EXAMPLE 7

N-[5-(2-{2-Amino-4-hydroxy-5,6,7,8-tetrahydropyrido[2,3-d]pyrimidin-6-yl}ethyl)thien-2-ylcarbonyl]-L-glutamic Acid

A solution of 100mg. of dimethyl N-[5-(2-{2-pivaloylamino-4-hydroxy-5,6,7,8-tetrahydropyrido[2,3-d]-pyrimidin-6-yl}ethyl)thien-2-ylcarbonyl]-L-glutamate in 15mL. of 1.0 N aqueous sodium hydroxide is stirred at room temperature for 120 hours and the pH then adjusted to 7.0 through the careful addition of 5.0 N hydrochloric acid. The solid which formed was collected by filtration and dried at 80°C to yield 61.5mg (76.8%) of N-[5-(2-{2-amino-4-hydroxy-5,6,7,8-tetrahydropyrido[2,3-d]pyrimidin-6-yl}ethyl)thien-2-ylcarbonyl]-L-glutamic acid, m.p. 226-240°C. (foaming and decomposition). NMR (DMSO-$d_6$ 300 MHz) delta: 9.80 (s, br, 1H), 8.45 (d, J=9 Hz, 1H), 7.62 (d, J=3 Hz, 1H), 6.87 (d, J=3 Hz, 1H), 6.30 (s, 1H), 6.04 (s, 2H), 4.28 (m, 1H), 3.12 (m, 1H), 2.84 (m, 3H), 2.36 (m, 1H), 2.02 (m, 1H), 1.83 (m, 2H), 1.57 (m, 3H).

Similarly prepared from dimethyl N-[5-(2-{2-pivaloylamino-4-hydroxy-5,6,7,8-tetrahydropyrido[2,3-d]-pyrimidin-6-yl}ethyl)fur-2-ylcarbonyl]-L-glutamate is N-[5-(2-{2-amino-4-hydroxy-5,6,7,8-tetrahydropyrido-[2,3-d]pyrimidin-6-yl}ethyl)fur-2-ylcarbonyl-L-glutamic acid. In a representative experiment, the fol-lowing physical constants were obtained for this compound: m.p. 204-230°C. (foaming). NMR (DMSO-$d_6$ 300 MHz) delta: 9.80 (s, br, 1H), 8.27 (d, J=9 Hz, 1H), 7.03 (d, J=4 Hz, 1H), 6.30 (s, 1H), 6.25 (d, J=4 Hz, 1H), 6.04

(s, 2H), 4.30 (m, 1H), 3.16 (m, 1H), 2.65 (m, 3H), 2.42 (m, 1H), 2.16 (t, J=7Hz, 2H), 2.01 (m, 1H), 1.84 (m, 2H), 1.57 (m, 3H).

N-[5-(2-{2-Amino-4-hydroxy-5,6,7,8-tetrahydropyrido[2,3-d]pyrimidin-6-yl}ethyl)thien-3-ylcarbonyl]-L-glutamic acid can be prepared similarly from dimethyl N-[5-(2-{2-pivaloylamino-4-hydroxy-5,6,7,8-tetrahydropyrido[2,3-d]pyrimidin-6-yl}ethyl)thien-3-ylcarbonyl]-L-glutamate.

EXAMPLE 8

The $IC_{50}$ in whole cell human leukemia cell lines, CCRF-CEM, of N-[5-(2-{2-amino-4-hydroxy-5,6,7,8-tetra-hydropyrido[2,3-d]pyrimidin-6-yl}ethyl)thien-2-ylcarbo-nyl]-L-glutamic acid is 0.0015 ug/mL. The $IC_{50}$ of N-[5-(2-{2-amino-4-hydroxy-5,6,7,8-tetrahydropyrido[2,3-d]pyrimidin-6-yl}ethyl)fur-2-ylcarbonyl]-L-glutamic acid is 0.010 ug/mL. The $IC_{50}$ of N-[4-(2-{2-amino-4-hydroxy-5,6,7,8-tetrahydropyrido[2,3-d]pyrimidin-6-yl}-ethyl)thien-2-ylcarbonyl]-L-glutamic acid is 0.0756 ug/mL.

The $IC_{50}$ in whole cell human leukemia cell lines, CCRF-CEM, of other compounds of this invention are as follows:

| $R^1$ | $R^2$ | $R^3$ | $R^4$ | ug/mL |
|---|---|---|---|---|
| OH | H | $-(CH_2)_2-$ | H | 0.023 |
| OH | H | $-(CH_2)_3-$ | H | 0.0075 |
| OH | H | $-(CH_2)_4-$ | H | 0.034 |
| OH | H | 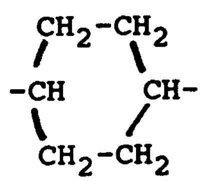 | H | 0.007 |

In vivo evaluation against various neoplasms produced the following values:

(a.) N-[5-(2-{2-amino-4-hydroxy-5,6,7,8-tetra-hydropyrido[2,3-d]pyrimidin-6-yl}ethyl)thien-2-yl-carbonyl]-L-glutamic acid

| 6C3HED lymphosarcoma tumors in mice: (IP, daily administration, one day) | | |
|---|---|---|
| Dose mg/kg | % Inhibition | Toxicity |
| 400 | 100 | 6/10 |
| 200 | 99 | 2/10 |
| 100 | 94 | 1/10 |
| 50 | 88 | 0/10 |

(b.) N-[5-(2-{2-amino-4-hydroxy-5,6,7,8-tetra-hydropyrido[2,3-d]pyrimidin-6-yl}ethyl)thien-2-yl-carbonyl]-L-glutamic acid

| 6C3HED lymphosarcoma tumors in mice: (IP, daily administration, 10 days) | | |
|---|---|---|
| Dose mg/kg | % Inhibition | Toxicity |
| 6.25 | toxic | 10/10 |
| 3.12 | 100 | 6/10 |
| 1.56 | 100 | 0/10 |
| 0.78 | 93 | 0/10 |

(c.) N-[5-(2-{2-amino-4-hydroxy-5,6,7,8-tetra-hydropyrido[2,3-d]pyrimidin-6-yl}ethyl)thien-2-yl-carbonyl]-L-glutamic acid

| B-16 Melanoma tumors in mice: (IP, daily administration, days 1, 7) | | |
|---|---|---|
| Dose mg/kg | % Inhibition | Toxicity |
| 200 | 93 | 4/10 |
| 100 | 73 | 3/10 |
| 50 | 38 | 0/9 |

(d.) N-[5-(2-{2-amino-4-hydroxy-5,6,7,8-tetrahydropyrido[2,3-d]pyrimidin-6-yl}ethyl)thien-2-yl-carbonyl]-L-glutamic acid

| C3H Mammary Adenocarcinoma tumors in mice: (IP, daily administration, days 1, 4, 7, 10) | | |
|---|---|---|
| Dose mg/kg | % Inhibition | Toxicity |
| 40 | 100 | 0/10 |
| 20 | 100 | 0/10 |
| 10 | 94 | 0/10 |
| 5 | 90 | 1/10 |
| 2.5 | 71 | 0/10 |

(e.) N-[5-(2-{2-amino-4-hydroxy-5,6,7,8-tetrahydropyrido[2,3-d]pyrimidin-6-yl}ethyl)thien-2-yl-carbonyl]-L-glutamic acid

| CA-755 Adenocarcinoma tumors in mice: (IP, daily administration, days 1, 4, 7, 10) | | |
|---|---|---|
| Dose mg/kg | % Inhibition | Toxicity |
| 40 | 83 | 0/10 |
| 20 | 78 | 0/10 |

(f.) N-[5-(2-{2-amino-4-hydroxy-5,6,7,8-tetrahydropyrido[2,3-d]pyrimidin-6-yl}ethyl)thien-2-yl-carbonyl]-L-glutamic acid

| CX-1 Colon Xenography in mice: (IP, daily administration, days 1, 4, 7, 10) | | |
|---|---|---|
| Dose mg/kg | % Inhibition | Toxicity |
| 40 | toxic | 10/10 |
| 20 | 91 | 9/10 |
| 10 | 94 | 6/10 |
| 5 | 90 | 3/10 |

(g.) N-[5-(2-{2-amino-4-hydroxy-5,6,7,8-tetrahydropyrido[2,3-d]pyrimidin-6-yl}ethyl)thien-2-yl-carbonyl]-L-glutamic acid

| LX-1 Lung Xenography in mice: (IP, daily administration, days 1, 4, 7, 10) | | |
|---|---|---|
| Dose mg/kg | % Inhibition | Toxicity |
| 40 | toxic | 8/8 |
| 20 | 100 | 4/8 |
| 10 | 98 | 0/8 |

(h.) N-[5-(2-{2-amino-4-hydroxy-5,6,7,8-tetrahydropyrido[2,3-d]pyrimidin-6-yl}ethyl)thien-2-yl-carbonyl]-L-glutamic acid

| MX-1 Mammary Xenography in mice: (IP, daily administration, days 1, 4, 7, 10) | | |
|---|---|---|
| Dose mg/kg | % Inhibition | Toxicity |
| 40 | toxic | 10/10 |
| 20 | toxic | 9/9 |
| 10 | 96 | 5/10 |
| 5 | 84 | 3/10 |

(i.) N-[5-(2-{2-amino-4-hydroxy-5,6,7,8-tetrahydropyrido[2,3-d]pyrimidin-6-yl}ethyl)fur-2-yl-carbonyl]-L-glutamic acid

| 6C3HED lymphosarcoma tumors in mice: (IP, daily administration, eight days) | | |
|---|---|---|
| Dose mg/kg | % Inhibition | Toxicity |
| 100 | 89 | 0/10 |
| 50 | 69 | 0/10 |
| 25 | 59 | 0/10 |

(j.) N-[5-(2-{2-amino-4-hydroxy-5,6,7,8-tetrahydropyrido[2,3-d]pyrimidin-6-yl}ethyl)fur-2-yl-carbonyl]-L-glutamic acid

| 6C3HED lymphosarcoma tumors in mice: (IP, daily administration, days 1, 4, 7) | | |
|---|---|---|
| Dose mg/kg | % Inhibition | Toxicity |
| 20 | 100 | 0/10 |
| 10 | 100 | 0/10 |
| 5 | 97 | 0/10 |
| 2.5 | 98 | 0/10 |
| 1.25 | 29 | 0/10 |
| 0.625 | 1 | 0/10 |

(k.) N-[4-(2-amino-4-hydroxy-5,6,7,8-tetrahydropyrido[2,3-d]pyrimidin-6-yl)butyryll]-L-glutamic acid

| 6C3HED lymphosarcoma tumors in mice: (IP, daily administration, eight days) | | |
|---|---|---|
| Dose mg/kg | % Inhibition | Toxicity |
| 50 | 98 | 1/10 |
| 25 | 88 | 0/10 |
| 12.5 | 62 | 0/10 |
| 6.25 | 11 | 0/10 |
| 3.12 | 0 | 0/10 |

(l.) N-{4-[2-(2-amino-4-hydroxy-5,6,7,8-tetrahydropyrido[2,3-d] pyrimidin-6-yl)ethyl]cyclohexylcarbonyl}-L-glutamic acid

| 6C3HED lymphosarcoma tumors in mice: (IP, daily administration, eight days) | | |
|---|---|---|
| Dose mg/kg | % Inhibition | Toxicity |
| 100 | 22 | 0/9 |
| 50 | 5 | 0/10 |
| 25 | 4 | 0/10 |
| 12.5 | 18 | 0/10 |

## Claims

1. A compound selected from the group consisting of a glutamic acid derivative of the formula:

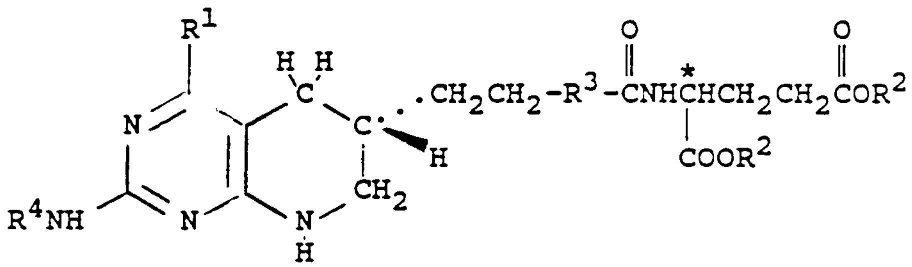

or

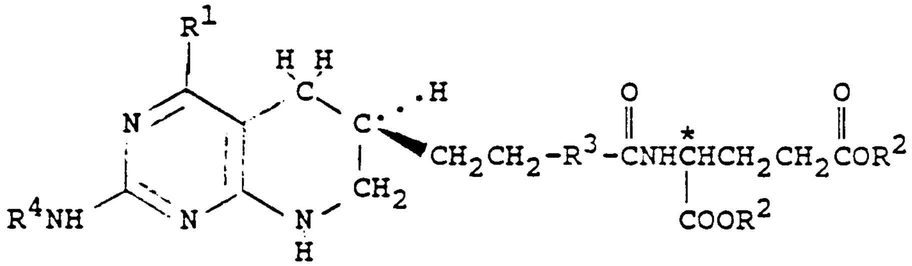

in which:

$R^1$ is -OH or -$NH_2$;

$R^2$ is hydrogen or a carboxy protecting group;

$R^3$ is a carbon-carbon bond, alkylene of 1 to 4 carbon atoms, cyclohexylene, or

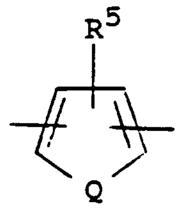

;

$R^4$ is hydrogen or an amino protecting group;

Q is -S- or -O-;

$R^5$ is hydrogen, chloro or fluoro; and

the configuration about the carbon atom designated * is S; and

the pharmaceutically acceptable salts thereof.

2. A compound according to claim 1 wherein $R^1$ is -OH and each of $R^2$ and $R^4$ is hydrogen.

3. A compound according to claim 1 or claim 2 wherein $R^3$ is methylene, ethylene, or trimethylene.

4. A compound according to claim 1, claim 2 or claim 3 which is (S,S)-N-[3-(2-amino-4-hydroxy-5,6,7,8-tetrahydropyrido[2,3-d]pyrimidin-6-yl)propioyl]-L-glutamic acid or (R,S)-N-[3-(2-amino-4-hydroxy-5,6,7,8-tetrahydropyrido-[2,3-d]pyrimidin-6-yl)propioyl]-L-glutamic acid.

5. A compound according to claim 1, claim 2 or claim 3 which is (S,S)-N-[4-(2-amino-4-hydroxy-5,6,7,8-tetrahydropyrido[2,3-d]pyrimidin-6-yl)butanoyl]-L-glutamic acid or (R,S)-N-[4-(2-amino-4-hydroxy-5,6,7,8-tetrahydropyrido-[2,3-d]pyrimidin-6-yl)butanoyl]-L-glutamic acid.

**6.** A compound according to claim 1, claim 2 or claim 3 which is (S,S)-N-[5-(2-amino-4-hydroxy-5,6,7,8-tetrahydropyrido[2,3-d]pyrimidin-6-yl)pentanoyl]-L-glutamic acid or (R,S)-N-[3-(2-amino-5-hydroxy-5,6,7,8-tetrahydropyrido-[2,3-d]pyrimidin-6-yl)pentanoyl]-L-glutamic acid.

**7.** A compound according to claim 1, claim 2 or claim 3 which is (S,S)-N-[6-(2-amino-4-hydroxy-5,6,7,8-tetrahydropyrido[2,3-d]pyrimidin-6-yl)hexanoyl]-L-glutamic acid or (R,S)-N-[3-(2-amino-6-hydroxy-5,6,7,8-tetrahydropyrido-[2,3-d]pyrimidin-6-yl)hexanoyl]-L-glutamic acid.

**8.** A compound according to claim 1 or claim 2 wherein $R^3$ is cyclohexa-1,4-diyl.

**9.** A compound according to claim 1, claim 2 or claim 8 which is (S,S)-N-[4-(2-{2-amino-4-hydroxy-5,6,7,8-tetrahydropyrido-[2,3-d]pyrimidin-6-yl}ethyl)cyclohex-1-ylcarbonyl]-L-glutamic acid or (R,S)-N-[4-(2-{2-amino-4-hydroxy-5,6,7,8-tetrahydropyrido[2,3-d]pyrimidin-6-yl}-ethyl)cyclohex-1-ylcarbonyl]-L-glutamic acid.

**10.** A compound according to claim 1 or claim 2 wherein $R^3$ is

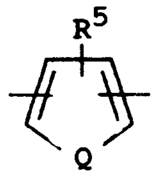

**11.** A compound according to claim 1, claim 2 or claim 10 wherein $R^5$ is hydrogen and Q is -S-.

**12.** A compound according to claim 1, claim 2, claim 10 or claim 11 which is (S,S)-N-[5-(2-{2-amino-4-hydroxy-5,6,7,8-tetrahydropyrido-[2,3-d]pyrimidin-6-yl}ethyl)thien-2-ylcarbony]-L-glutamic acid or (R,S)-N-[5-(2-{2-amino-4-hydroxy-5,6,7,8-tetrahydropyrido[2,3-d]pyrimidin-6-yl}-ethyl)thien-2-ylcarbony]-L-glutamic acid.

**13.** A compound according to claim 1, claim 2 or claim 10 wherein $R^5$ is hydrogen and Q is -O-.

**14.** A compound according to claim 1, claim 2 or claim 10 which is (S,S)-N-[5-(2-{2-amino-4-hydroxy-5,6,7,8-tetrahydropyrido-[2,3-d]pyrimidin-6-yl}ethyl)furan-2-ylcarbony]-L-glutamic acid or (R,S)-N-[5-(2-{2-amino-4-hydroxy-5,6,7,8-tetrahydropyrido[2,3-d]pyrimidin-6-yl}-ethyl)furan-2-ylcarbony]-L-glutamic acid. use in the manufacture of a medicament for

**15.** The use in the manufacture of a medicament for combatting neoplastic growth in a mammal of an effective amount of a compound according to any preceding claim.

**16.** A pharmaceutical composition for combatting neoplastic growth in a mammal which comprises an amount of a compound according to any of claims 1 - 14 which upon administration to the mammal in a single or multiple dose regimen is effective to combat said growth, in combination with a pharmaceutically acceptable carrier.

**17.** Process for the preparation of a compound as claimed in any of claims 1 - 14. selected from the group consisting of a glutamic acid derivative of the formula:

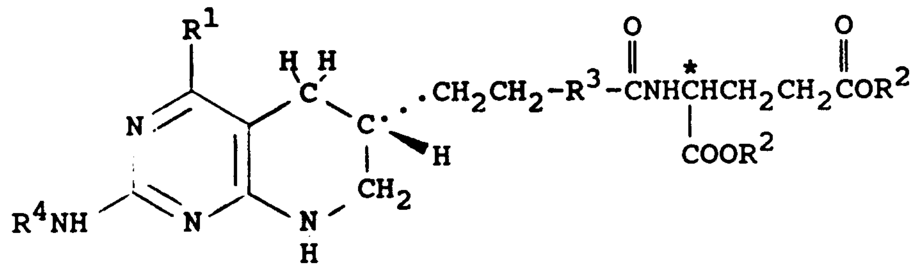

or

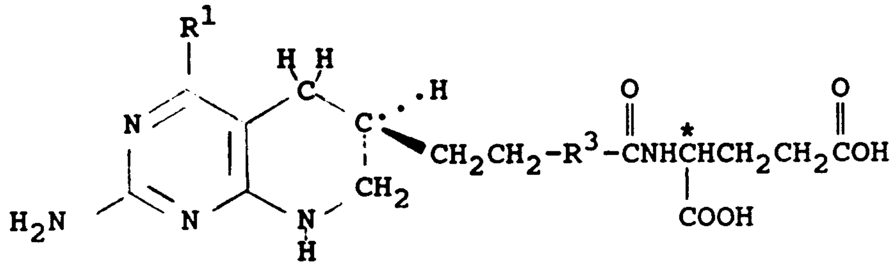

in which:

$R^1$ is -OH or $-NH_2$;

$R^3$ is a carbon-carbon bond, alkylene of 1 to 4 carbon atoms, cyclohexylene, or

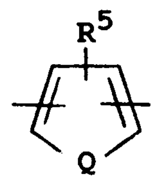

;

Q is -S- or -O-;

$R^5$ is hydrogen, chloro or fluoro; and

the configuration about the carbon atom designated * is S; and

the pharmaceutically acceptable salts thereof which comprises hydrolysing a compound of the formula:

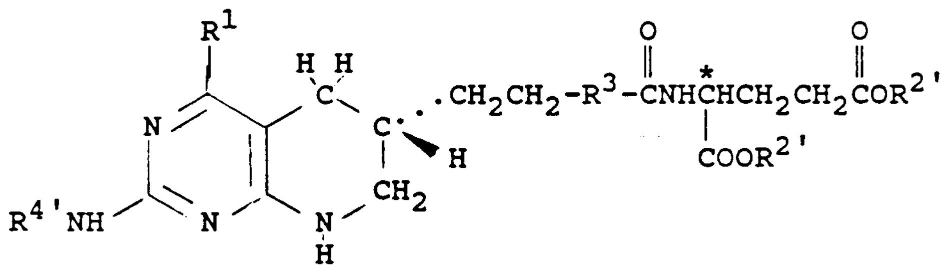

or

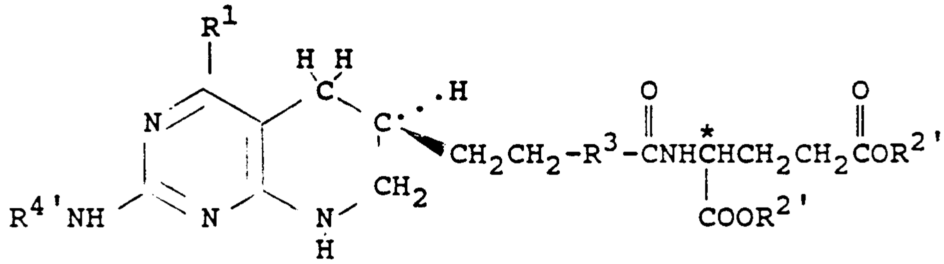

or a mixture thereof,
in which:

$R^1$, $R^3$, Q, and $R^5$ are as defined herein;
$R^{2'}$ is hydrogen or a carboxy protecting group;
$R^{4'}$ is hydrogen or an amino protecting group; and
the configuration about the carbon atom designated * is S
at least one of $R^{2'}$ and $R^{4'}$ being other than hydrogen.

**Patentansprüche**

**1.** Verbindung ausgewählt aus der Gruppe, bestehend aus Glutaminsäurederivaten der Formel:

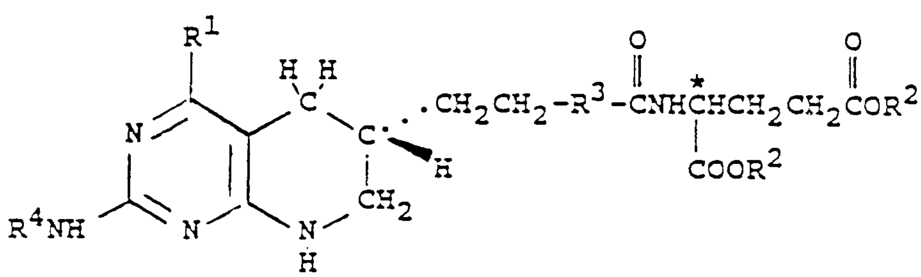

or

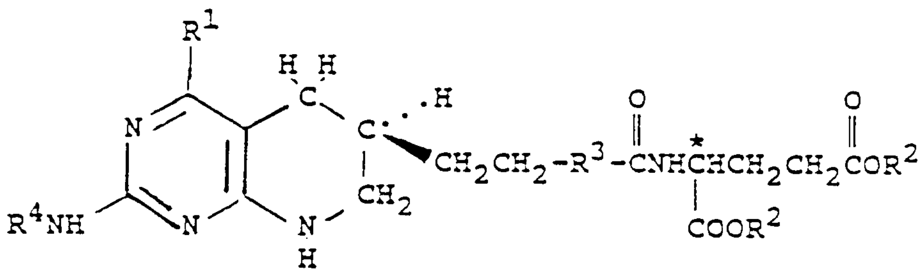

in der:
$R^1$ -OH oder $-NH_2$ ist;

$R^2$ Wasserstoff oder eine Carboxyschutzgruppe ist;
$R^3$ eine Kohlenstoff-Kohlenstoff-Bindung, eine Alkylengruppe mit 1 bis 4 Kohlenstoffatomen, Cyclohexylen oder

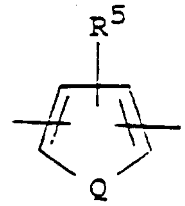

ist;
$R^4$ Wasserstoff oder eine Aminoschutzgruppe ist;
Q -S- oder -O- ist;
$R^5$ Wasserstoff, Chlor oder Fluor; und
die Konfiguration um das Kohlenstoffatom, das mit einem * gekennzeichnet ist, ist eine S-Konfiguration; und
deren pharmazeutisch akzeptablen Salze.

**2.** Verbindung nach Anspruch 1, bei der $R^1$ -OH und ein jedes $R^2$ und $R^4$ Wasserstoff ist.

**3.** Verbindung nach Anspruch 1 oder Anspruch 2, bei der $R^3$ eine Methylen-, Ethylen- oder Trimethylengruppe ist.

**4.** Eine Verbindung nach Anspruch 1, Anspruch 2 oder Anspruch 3, die (S,S)-N-[3-(2-Amino-4-hydroxy-5,6,7,8-tetrahydropyrido[2,3-d]-pyrimidin-6-yl)propioyl]-L-glutaminsäure oder (R,S)-N-[3-(2-Amino-4-hydroxy-5,6,7,8-tetrahydropyrido[2,3-d]-pyrimidin-6-yl)propioyl]-L-glutaminsäure ist.

**5.** Eine Verbindung nach Anspruch 1, Anspruch 2 oder Anspruch 3, die (S,S)-N-[4-(2-Amino-4-hydroxy-5,6,7,8-tetrahydropyrido[2,3-d]-pyrimidin-6-yl)butanoyl]-L-glutaminsäure oder (R,S)-N-[4-(2-Amino-4-hydroxy-5,6,7,8-tetrahydropyrido[2,3-d]-pyrimidin-6-yl)butanoyl]-L-glutaminsäure ist.

**6.** Eine Verbindung nach Anspruch 1, Anspruch 2 oder Anspruch 3, die (S,S)-N-[5-(2-Amino-4-hydroxy-5,6,7,8-tetrahydropyrido[2,3-d]-pyrimidin-6-yl)pentanoyl]-L-glutaminsäure oder (R,S)-N-[3-(2-Amino-4-hydroxy-5,6,7,8-tetrahydropyrido[2,3-d]-pyrimidin-6-yl)pentanoyl]-L-glutaminsäure ist.

**7.** Eine Verbindung nach Anspruch 1, Anspruch 2 oder Anspruch 3, die (S,S)-N-[6-(2-Amino-4-hydroxy-5,6,7,8-tetrahydropyrido[2,3-d]-pyrimidin-6-yl)hexanoyl]-L-glutaminsäure oder (R,S)-N-[3-(2-Amino-4-hydroxy-5,6,7,8-tetrahydropyrido[2,3-d]-pyrimidin-6-yl)hexanoyl]-L-glutaminsäure ist.

**8.** Verbindung nach Anspruch 1 oder Anspruch 2, bei der $R^3$ Cyclohexa-1,4-diyl ist.

**9.** Eine Verbindung nach Anspruch 1, Anspruch 2 oder Anspruch 8, die (S,S)-N-[4-(2-{2-Amino-4-hydroxy-5,6,7,8-tetrahydropyrido[2,3-d]-pyrimidin-6-yl}ethyl)cyclohex-1-ylcarbonyl]-L-glutaminsäure oder (R,S)-N-[4-(2-{2-Amino-4-hydroxy-5,6,7,8-tetrahydropyrido[2,3-d]-pyrimidin-6-yl}ethyl)cylohex-1ylcarbonyl]-L-glutaminsäure ist.

**10.** Verbindung nach Anspruch 1 oder Anspruch 2, bei der $R^3$

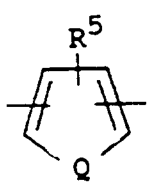

ist.

**11.** Verbindung nach Anspruch 1, Anspruch 2 oder Anspruch 10, bei der $R^5$ Wasserstoff und Q -S- ist.

**12.** Eine Verbindung nach Anspruch 1, Anspruch 2, Anspruch 10 oder Anspruch 11, die (S,S)-N-[5-(2-{2-Amino-4-hydroxy-5,6,7,8-tetrahydropyrido[2,3-d]-pyrimidin-6-yl}ethyl)thien-2-ylcarbonyl]-L-glutaminsäure oder (R,S)-N-[5-(2-{2-Amino-4-hydroxy-5,6,7,8-tetrahydropyrido[2,3-d]-pyrimidin-6-yl}ethyl)thien-2-ylcarbonyl]-L -glutaminsäure ist.

**13.** Verbindung nach Anspruch 1, Anspruch 2 oder Anspruch 10, bei der $R^5$ Wasserstoff und Q -O- ist.

**14.** Eine Verbindung nach Anspruch 1, Anspruch 2 oder Anspruch 10, die (S,S)-N-[5-(2-{2-Amino-4-hydroxy-5,6,7,8-tetrahydropyrido[2,3-d]-pyrimidin-6-yl}ethyl)furan-2-ylcarbonyl]-L-glutaminsäure oder (R,S)-N-[5-(2-{2-Amino-4-hydroxy-5,6,7,8-tetrahydropyrido[2,3-d]-pyrimidin-6-yl}ethyl)furan-2-ylcarbonyl]-L-glutaminsäure ist.

**15.** Verwendung bei der Herstellung eines Medikaments zur Bekämpfung des neoplatischen Wachstums in Mammalia mit einer effektiven Menge einer Verbindung nach irgendeinem vorherigen Anspruch.

**16.** Pharmazeutische Zusammensetzung zur Bekämpfung des neoplastischen Wachstums in Mammalia, die einen Anteil einer Verbindung nach irgendeinem der Ansprüche 1 bis 14 enthält, die nach Administration an Mammalia in Einzel- oder Mehrfachdosierungen effektiv ist, das besagte Wachstum in Kombination mit einem pharmazeutisch akzeptablen Trägermaterial zu bekämpfen.

**17.** Verfahren zur Herstellung einer Verbindung nach irgendeinem der Ansprüche 1 bis 14 ausgewählt aus der Gruppe, bestehend aus Glutaminsäurederivaten der Formel:

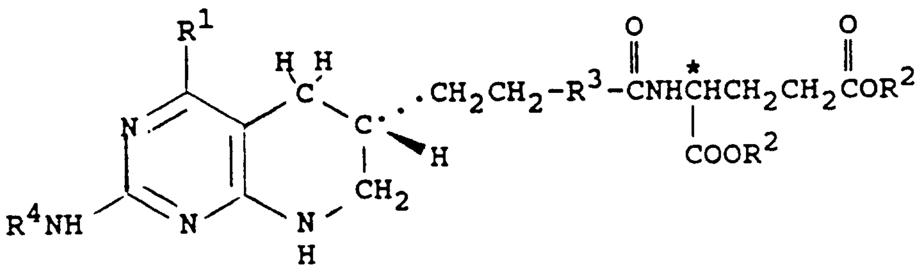

or

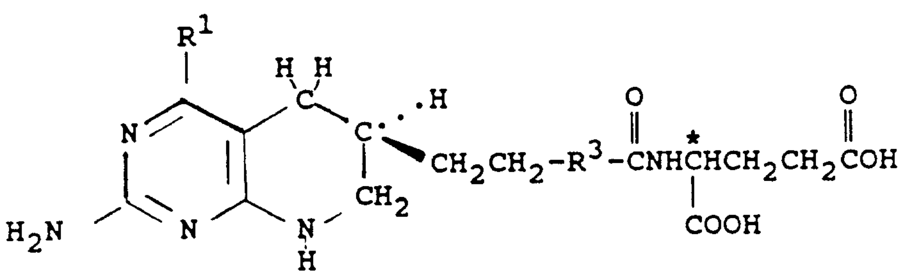

in der:

$R^1$ -OH oder $-NH_2$ ist;

$R^3$ eine Kohlenstoff-Kohlenstoff-Bindung, eine Alkylengruppe mit 1 bis 4 Kohlenstoffatomen, Cyclohexylen oder

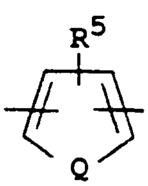

ist;
Q -S- oder -O- ist;
$R^5$ Wasserstoff, Chlor oder Fluor; und
die Konfiguration um das Kohlenstoffatom, das mit einem * gekennzeichnet ist, ist eine S-Konfiguration; und
deren pharmazeutisch akzeptablen Salze,
das die Hydrolyse einer Verbindung der Formel:

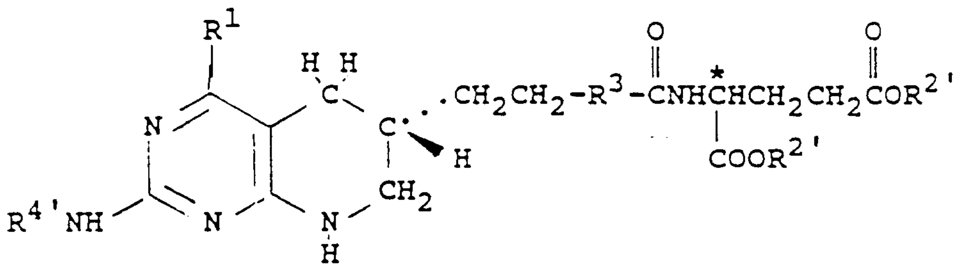

or

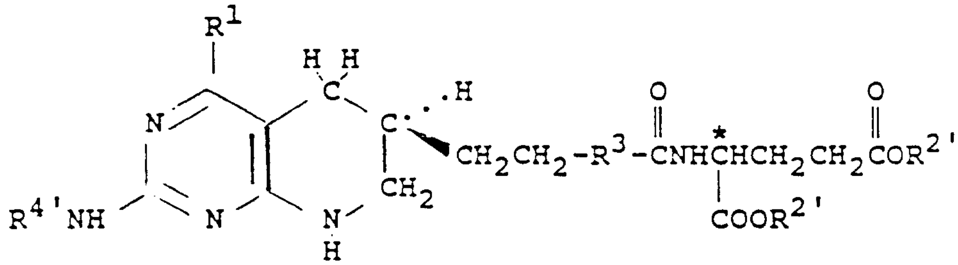

oder eine Mischung davon umfaßt,
in der:
$R^1$, $R^3$, Q udn $R^5$ wie oben definiert sind;
$R^2$ Wasserstoff oder eine Carboxyschutzgruppe ist;
$R^4$ Wasserstoff oder eine Aminoschutzgruppe ist; und die Konfiguration des Kohlenstoffatoms, das mit * gekennzeichnet ist, ist eine S-Konfiguration
wenigstens eines von $R^{2'}$ und $R^{4'}$ anders als Wasserstoff ist.

## Revendications

1. Composé choisi dans le groupe constitué d'un dérivé d'acide glutamique de formule :

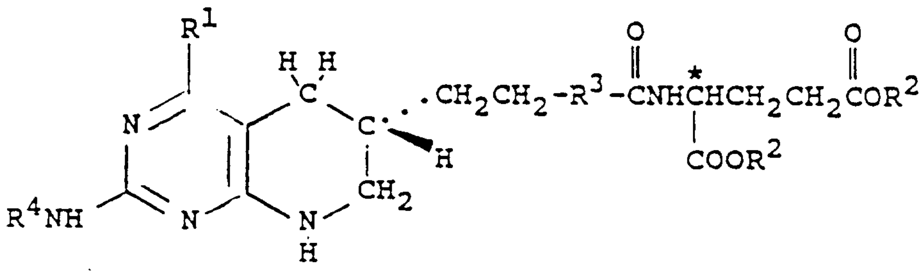

ou

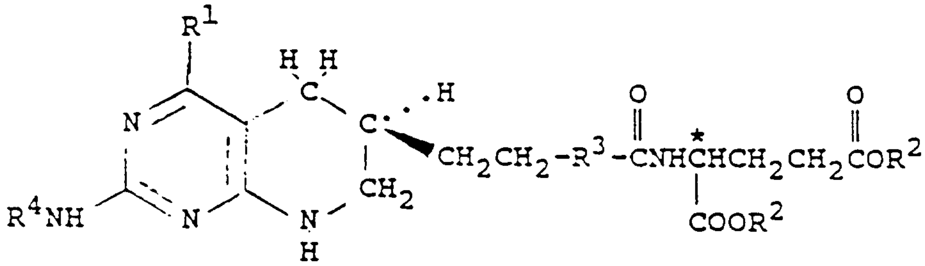

dans laquelle

$R^1$ représente -OH ou $-NH_2$ ;

$R^2$ est de l'hydrogène ou un groupe protégeant la fonction carboxy ;

$R^3$ est une liaison carbone-à-carbone, un groupe alkylène de 1 à 4 atomes de carbone, cyclohexylène ou

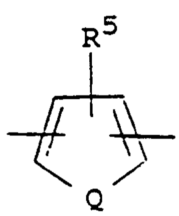

;

$R^4$ est de l'hydrogène ou un groupe protégeant la fonction amino ;

Q représente -S- ou -O- ;

$R^5$ est de l'hydrogène, un radical chloro ou fluoro ; et

la configuration autour de l'atome de carbone marqué d'un astérisque est la configuration S ; et ses sels pharmaceutiquement acceptables.

2. Composé suivant la revendication 1, dans lequel $R^1$ est un groupe -OH et chacun de $R^2$ et $R^4$ est de l'hydrogène.

3. Composé suivant la revendication 1 ou la revendication 2, dans lequel $R^3$ est un groupe méthylène, éthylène ou triméthylène.

4. Composé suivant la revendication 1, la revendication 2 ou la revendication 3, qui est l'acide (S,S)-N-[3-(2-amino-4-hydroxy-5,6,7,8-tétrahydropyrido[2,3-d]-pyrimidine-6-yl)propionyl]-L-glutamique ou l'acide (R,S)-N-[3-(2-amino-4-hydroxy-5,6,7,8-tétrahydropyrido-[2,3-d]-pyrimidine-6-yl)propionyl ]-L-glutamique.

5. Composé suivant la revendication 1, la revendication 2 ou la revendication 3, qui est l'acide (S,S)-N-[4-(2-amino-4-hydroxy-5,6,7,8-tétrahydropyrido[2,3-d]-pyrimidine-6-yl)butanoyl]-L-glutamique ou l'acide (R,S)-N-[4-(2-amino-4-hydroxy-5,6,7,8-tétrahydropyrido-[2,3-d]pyrimidine-6-yl)butanoyl]-L -glutamique.

6. Composé suivant la revendication 1, la revendication 2 ou la revendication 3, qui est l'acide (S,S)-N-[5-(2-amino-4-hydroxy-5,6,7,8-tétrahydropyrido[2,3-d]-pyrimidine-6-yl)pentanoyl]-L-glutamique ou l'acide (R,S)-N-[3-(2-amino-5-hydroxy-5,6,7,8-tétrahydropyrido-[2,3-d]pyrimidine-6-yl)pentanoyl] -L-glutamique.

7. Composé suivant la revendication 1, la revendication 2 ou la revendication 3, qui est l'acide (S,S)-N-[6-(2-amino-4-hydroxy-5,6,7,8-tétrahydropyrido[2,3-d]-pyrimidine-6-yl)hexanoyl]-L-glutamique ou l'acide (R,S)-N-[3-(2-amino-6-hydroxy-5,6,7,8-tétrahydropyrido-[2,3-d]pyrimidine-6-yl)hexanoyl]-L -glutamique.

8. Composé suivant la revendication 1 ou la revendication 2, dans lequel $R^3$ est le groupe cyclohexa-1,4-diyle.

9. Composé suivant la revendication 1, la revendication 2 ou la revendication 8, qui est l'acide (S,S)-N-[4-(2-{2-amino-4-hydroxy-5,6,7,8-tétrahydropyrido-[2,3-d]pyrimidine-6-yl}éthyl)cyclohex-1-ylcarbonyl]-L-glutamique ou l'acide (R,S)-N-[4-(2-{2-amino-4-hydroxy-5,6,7,8-tétrahydropyrido[2,3-d]pyrimidine-6-yl}-éthyl)cyclohex-1-yl-carbonyl]-L-glutamique.

10. Composé suivant la revendication 1 ou la revendication 2, dans lequel $R^3$ est un groupe

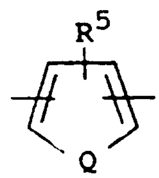

11. Composé suivant la revendication 1, la revendication 2 ou la revendication 10, dans lequel $R^5$ est l'hydrogène et Q représente -S-.

12. Composé suivant la revendication 1, la revendication 2, la revendication 10 ou la revendication 11, qui est l'acide (S,S)-N-[5-(2-{2-amino-4-hydroxy-5,6,7,8-tétrahydropyrido-[2,3-d]pyrimidine-6-yl}éthyl)thién-2-ylcarbonyl]-L-glutamique ou l'acide (R,S)-N-[5-(2-{2-amino-4-hydroxy-5,6,7,8-tétrahydropyrido[2,3-d]-pyrimidine-6-yl}-éthyl)thién-2-ylcarbonyl]-L-glutamique.

13. Composé suivant la revendication 1, la revendication 2 ou la revendication 10, dans lequel $R^5$ est l'hydrogène et Q représente -O-.

14. Composé suivant la revendication 1, la revendication 2 ou la revendication 10, qui est l'acide (S,S)-N-[5-(2-{2-amino-4-hydroxy-5,6,7,8-tétrahydropyrido-[2,3-d]pyrimidine-6-yl}éthyl)furanne-2-ylcarbonyl]-L-glutamique ou l'acide (R,S)-N-[5-(2-{2-amino-4-hydroxy-5,6,7,8-tétrahydropyrido[2,3-d]pyrimidine-6-yl}-éthyl)furanne-2-ylcarbonyl]-L-glutamique.

15. Utilisation, dans la préparation d'un médicament destiné à combattre une croissance néoplasique chez un mammifère, d'une quantité efficace d'un composé suivant l'une quelconque des revendications précédentes.

16. Composition pharmaceutique destinée à combattre une croissance néoplasique chez un mammifère, qui comprend une quantité d'un composé suivant l'une quelconque des revendications 1 à 14 qui, par administration à un mammifère selon une posologie impliquant une dose unique ou multiple, est efficace pour combattre ladite croissance, en association avec un support acceptable du point de vue pharmaceutique.

17. Procédé de production d'un composé suivant l'une quelconque des revendications 1 à 14 choisi dans le groupe comprenant un dérivé d'acide glutamique de formule

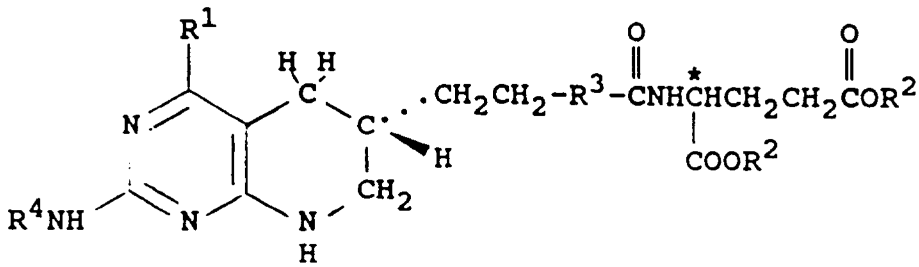

ou

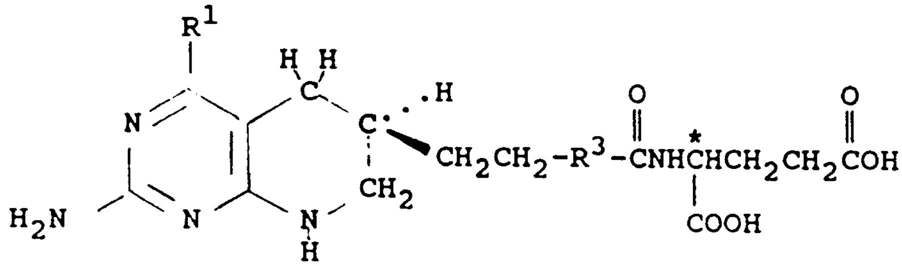

dans laquelle

$R^1$ représente -OH ou $-NH_2$ ;

$R^3$ est une liaison carbone-à-carbone, un groupe alkylène de 1 à 4 atomes de carbone, cyclohexylène ou

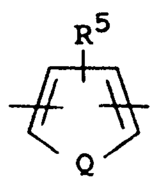

;

Q représente -S- ou -O- ;

$R^5$ est de l'hydrogène, un radical chloro ou fluoro ; et

la configuration autour de l'atome de carbone désigné par un astérisque est la configuration S ; et de leurs sels acceptables du point de vue pharmaceutique, qui comprend l'hydrolyse d'un composé de formule :

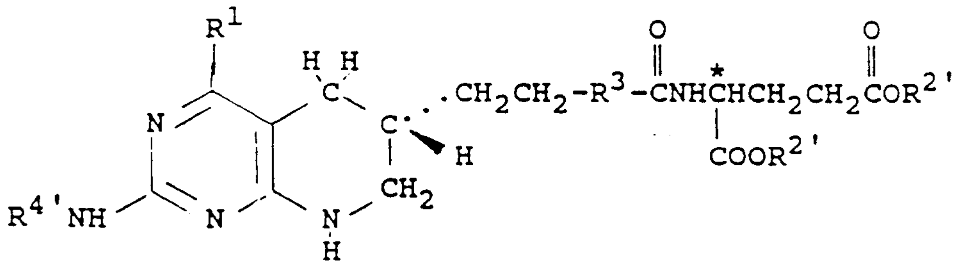

ou

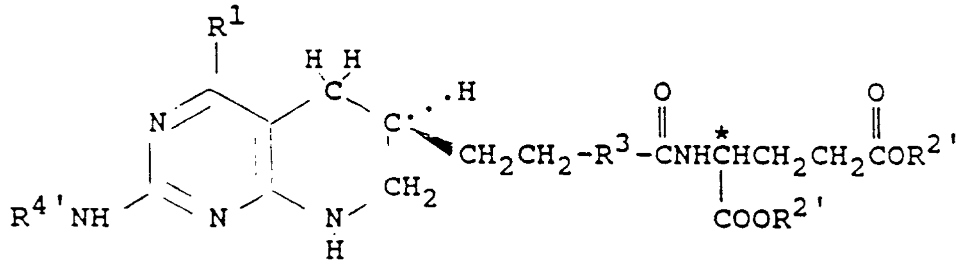

ou d'un mélange de tels composés, formule dans laquelle :

$R^1$, $R^3$, Q et $R^5$ sont tels que définis ci-dessus ;

$R^{2'}$ est de l'hydrogène ou un groupe protégeant la fonction carboxy ;

$R^{4'}$ est de l'hydrogène ou un groupe protégeant la fonction amino ; et

la configuration autour de l'atome de carbone désigné par un astérisque est la configuration S, l'un au moins de $R^{2'}$ et $R^{4'}$ représentant autre chose que de l'hydrogène.